(19) Europäisches Patentamt
European Patent Office
Office européen des brevets

(11) **EP 3 517 687 B1**

(12) # EUROPÄISCHE PATENTSCHRIFT

(45) Veröffentlichungstag und Bekanntmachung des Hinweises auf die Patenterteilung:
**05.08.2020 Patentblatt 2020/32**

(51) Int Cl.:
**E02D 1/02** *(2006.01)*  **E02D 3/054** *(2006.01)*
**E02D 7/18** *(2006.01)*

(21) Anmeldenummer: **18153596.4**

(22) Anmeldetag: **26.01.2018**

(54) **VERFAHREN ZUR VERDICHTUNGSERFASSUNG UND -STEUERUNG BEIM VERDICHTEN EINES BODENS MITTELS TIEFENRÜTTLER**

METHOD FOR COMPACTION DETECTION AND CONTROL WHEN COMPACTING SOIL USING DEEP VIBRATOR

PROCÉDÉ DE DÉTECTION ET DE COMMANDE DE COMPACTAGE LORS DU COMPACTAGE D'UN SOL AU MOYEN D'UN VIBREUR EN PROFONDEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**31.07.2019 Patentblatt 2019/31**

(73) Patentinhaber: **Keller Holding GmbH**
**63067 Offenbach/Main (DE)**

(72) Erfinder:
• **Nagy, Peter**
**1110 Wien (AT)**
• **Adam, Dietmar**
**2340 Mödling (AT)**
• **Adam, Christoph**
**6020 Innsbruck (AT)**

• **Schmitter, Maximilian**
**6365 Kirchberg in Tirol (AT)**
• **Kopf, Fritz**
**1140 Wien (AT)**
• **Strauch, Gerhard**
**1130 Wien (AT)**
• **Freitag, Peter**
**2601 Eggendorf / Siedlung Maria Theresia (AT)**

(74) Vertreter: **Neumann Müller Oberwalleney & Partner**
**Patentanwälte**
**Overstolzenstraße 2a**
**50677 Köln (DE)**

(56) Entgegenhaltungen:
EP-A1- 1 016 759        EP-A2- 0 584 680
DE-A1-102014 019 139    DE-C1- 19 928 692
DE-U1-202016 107 232

**Beschreibung**

Die Erfindung betrifft ein Verfahren zur Verdichtungserfassung und -steuerung beim Verdichten eines Bodens mittels eines Tiefenrüttlers sowie ein entsprechendes Verfahren zur Verbesserung eines Baugrundes mittels eines Tiefenrüttlers, das auf Basis von Daten aus dem Verfahren zur Verdichtungserfassung und -steuerung durchgeführt wird.

Zur Tiefenverdichtung locker gelagerter Böden werden Tiefenrüttler verwendet, welche durch eine rotierende Unwucht dynamisch angeregt werden und mit dem umgebenden, zu verdichtenden Boden ein dynamisches Interaktionssystem bildet. Anhand von Messungen am Rüttler können die dynamische Anregung, insbesondere Unwucht und deren Lage, sowie die Rüttlerbewegung bestimmt und daraus die dynamischen Eigenschaften des umgebenden Bodens ermittelt werden. Tiefenrüttler können in verschiedenen Verfahren zur Baugrundverbesserung eingesetzt werden, die beispielsweise in dem Prospekt "Die Tiefenrüttelverfahren" (Prospekt 10-02D) der Anmelderin beschrieben sind.

Das Verdichten des Bodens erfolgt in der Regel dadurch, dass der dynamisch angeregte Rüttler an einem Verdichtungspunkt gegebenenfalls unter Hilfe von Wasserspülung in den Boden bis zur gewünschten Verdichtungstiefe eindringt und dann entweder in Stufen gezogen und jeweils bis zu einem Abbruchkriterium gehalten wird oder Stopfbewegungen in einem aufwärtsbewegten Pilgerschrittverfahren durchführt. Flächige Verdichtung erfolgt durch die Bearbeitung vieler Verdichtungspunkte in einem Raster. Aus der WO 2012/171527 A1 sind ein Verfahren zur Bodensondierung und ein Verfahren zum Herstellen von Materialsäulen im Boden nach einer Bodensondierung bekannt. Das Verfahren umfasst die Schritte: Einfahren einer Rüttleranordnung bis zu einer vorbestimmten Tiefe in den Boden; Ermitteln eines Bodenprofils des Bodens beim Einfahren der Rüttleranordnung, wobei das Ermitteln des Bodenprofils das Messen wenigstens eines Betriebsparameters der Rüttleranordnung beim Einfahren in den Boden umfasst und wobei das Bodenprofil jeweils einen Bodenparameter für wenigstens zwei unterschiedliche Bodentiefen umfasst. Als Betriebsparameter können die Leistungsaufnahme des Rüttlermotors beim Einfahren der Rüttleranordnung in den Boden, die Geschwindigkeit, mit der die Rüttleranordnung in den Boden einfährt, oder eine Schwingungsamplitude der Spitze der Rüttleranordnung gemessen werden.

Aus der DE 199 28 692 C1 ist ein Verfahren zur online-Verdichtungskontrolle eines Bodens beim Einsatz eines Tiefenrüttlers bekannt. Das Verfahren sieht vor, dass zur gemessenen Eindringtiefe des Tiefenrüttlers der Verkippungswinkel zur vertikalen Nullachse, der Vorlaufwinkel der Unwucht gegenüber dem Rüttlermantel und mindestens eine horizontale Auslenkung zwischen der Rüttlerspitze und des Rüttlergelenks über einen oder mehrere Sensoren, die an oder in der Rüttlerspitze befestigt sind, direkt oder indirekt erfasst wird. Aus diesen erfassten Sensordaten werden bodendynamische Kennwerte des Systems Rüttler-Boden wie Dämpfung und Federsteifigkeit berechnet und hieraus die momentane Lagerungsdichte des umgebenden Bodens ermittelt. Dieser für die Lagerungsdichte ermittelte Wert wird ständig mit einem vorgegebenen Zielwert für die Lagerungsdichte des Bodens verglichen, und der Rüttelvorgang wird so lange weitergeführt, bis der Zielwert der Lagerungsdichte des Bodens erreicht ist.

Aus der DE 101 46 342 A1 ist ein Verfahren zur Ermittlung der Lagerungsdichte des Bodens während der Bodenverbesserung mittels Rütteldruckverdichtung bekannt. Dabei wird ein Tiefenrüttler mit einer um eine vertikale Achse rotierenden Unwucht an einem Gestänge hängend, gegebenenfalls unter Zuführung von Wasser und/oder Luft, in den Boden eingerüttelt, in einzelnen Höhenschritten gezogen und in Rüttelintervallen in einzelnen Tiefenlagen rüttelnd gehalten. Die Lagerungsdichte wird aus der Amplitude des Rüttlers unter Berücksichtigung spannungsabhängiger Bodenkennwerte mittels einer Rechner- oder Prozessoreinheit berechnet.

Aus der EP15 169 61 B1 wie auch der DE 10 2014 019 139 A1 sind ein Verfahren zur Ermittlung eines Maßes für die Bodensteifigkeit eines verdichteten beziehungsweise zu verdichtenden Bodenbereichs sowie eine Bodenverdichtungsvorrichtung bekannt. Die Bodensteifigkeit wird aus in den Boden eingeleiteten Schwingungen, aus den Maschinenparametern der Bodenverdichtungsvorrichtung und aus der zeitlichen Lage der Bodenverdichtungskraft ermittelt. Der vorliegenden Erfindung liegt die Aufgabe zugrunde, ein Verfahren zur online-Verdichtungserfassung und -steuerung vorzuschlagen, das eine effiziente Bodenverdichtung mittels eines Tiefenrüttlers ermöglicht. Die Aufgabe besteht ferner darin, ein entsprechendes Verfahren zur Verbesserung eines Baugrundes mittels eines Tiefenrüttlers vorzuschlagen, das auf Basis eines solchen Verfahrens zur online-Verdichtungserfassung und -steuerung durchgeführt wird.

Zur Lösung wird ein Verfahren zur Verdichtungserfassung und -steuerung beim Verdichten eines Bodens mittels eines Tiefenrüttlers vorgeschlagen, wobei der Tiefenrüttler eine in einem Rüttlergehäuse drehend antreibbare Unwucht sowie zumindest einen Sensor aufweist, mit den Schritten: Einbringen des Tiefenrüttlers in den Boden bis zu einer gewünschten Endtiefe ($T_m$); Verdichten des Bodens mittels des Tiefenrüttlers in Verdichtungsschritten, wobei während des Verdichtens zu einer jeweils gemessenen Tiefe ($T$) der Vorlaufwinkel ($\varphi$) der Unwucht sowie die Schwingungsamplitude ($A$) des Tiefenrüttlers ermittelt werden; während zumindest eines Verdichtungsschritts, Erfassen eines Bodensteifigkeitsverlaufs aus über der Zeit ($t$) auf Basis zumindest des Vorlaufwinkels ($\varphi$) und der Schwingungsamplitude ($A$) ermittelten Bodensteifigkeitswerten ($k$); Ermitteln eines ersten Bodensteifigkeitswerts ($k1$) und eines zweiten Bodensteifigkeitswerts ($k2$) aus dem Bodensteifigkeitsverlauf, für die gilt, dass eine Steigerungsrate ($k'2$) des zweiten Bodensteifigkeitswerts ($k2$) eine Steigerungsrate ($k'1$) des ersten Bodensteifigkeitswerts ($k1$) um einen definierten Faktor überschreitet; Berechnen eines Übergangs-Bodensteifigkeitswerts ($k12$), der zwischen dem ersten Bodensteifigkeitswert ($k1$) und dem zweiten Bodensteifigkeitswert (k2) liegt; und Speichern des in dem jeweiligen Verdichtungsschritt erfassten Übergangs-Bodensteifigkeitswerts (k12) zur zugehörigen Tiefe (T). Auf Basis zumindest einer Teilzahl der über der Tiefe (T) erfassten Übergangs-Bodensteifigkeitswerte (k12) kann ein Bodensteifigkeitsprofils erstellt werden.

Durch das Verdichten des Bodens um den Rüttler erhöht sich dessen Steifigkeit. Die Bestimmung der Bodensteifigkeit erfolgt durch begleitende Messungen während des Verdichtungsvorganges. Hiermit ist gemeint, dass die jeweiligen physikalischen Größen und/oder Kennwerte über der Zeit erfasst beziehungsweise berechnet werden. Dies kann fortlaufend beziehungsweise in regelmäßigen oder unregelmäßigen Zeitintervallen erfolgen. Zum Ermitteln der Bodensteifigkeitswerte über der Tiefe der zu erstellenden Verdichtungskörper wird der Rüttler ausgehend von der Endtiefe stufenweise in einzelnen Verdichtungsschritten von unten nach oben gezogen. Die einzelnen Verdichtungsschritte beziehen sich jeweils auf eine zugehörige Verdichtungstiefe, auch als Verdichtungspunkt bezeichnet, auf die der Rüttler gebracht wird, um diesen Bodenbereich zu verdichten. Für zumindest eine Teilzahl, insbesondere den größten Teil, vorzugsweise alle Verdichtungstiefen wird die Bodensteifigkeit über der Zeit ermittelt. Die ermittelte Entwicklung der Bodensteifigkeit kann zur Dokumentation des Verdichtungserfolges, zur Optimierung der Prozessparameter, des Verdichtungsablaufes, des gewählten Verdichtungs-Rasters oder zur automatischen Regelung des Rüttlers verwendet werden.

Ein Vorteil des Verfahrens zur online-Verdichtungskontrolle ist, dass zuverlässig erkennbar wird, wann der Boden nicht mehr oder kaum noch verdichtet wird. Dies erfolgt durch Ermittlung der Bodensteifigkeit über der Zeit innerhalb einer Verdichtungstiefe während des Verdichtungsprozesses. Aus zwei oder mehr ermittelten Bodensteifigkeitswerten kann die Steigung der Bodensteifigkeit abgeleitet werden. Es ist vorgesehen, dass das Bodensteifigkeitsprofil für die Herstellung weiterer Verdichtungskörper verwendet wird. Dabei kann der Verdichtungsprozess beziehungsweise das Einrütteln in einer bestimmten Tiefe beendet werden, wenn die ermittelte Bodensteifigkeit plötzlich zunimmt, das heißt die ermittelte Bodensteifigkeit über der Zeit stärker steigt als bei zuvor ermittelten Bodensteifigkeiten, beziehungsweise der zur jeweiligen Tiefe zugehörige Übergangs-Bodensteifigkeitswert erreicht ist.

Es wird insbesondere davon ausgegangen, dass die Steigerung der Bodensteifigkeit - solange der Boden weiter verdichtet wird - zumindest im Wesentlichen konstant ist. Mit der Formulierung "im Wesentlichen konstant" soll vorliegend insbesondere berücksichtigt werden, dass Messungenauigkeiten und Bodenunregelmäßigkeiten zu Abweichungen führen können. Wenn der Boden weitestgehend verdichtet ist, führt ein weiteres Rütteln zu einem erkennbaren Anstieg der Bodensteifigkeit. Die Funktion der über der Zeit ermittelten Bodensteifigkeit vollzieht einen erkennbaren Knick. Dies lässt sich insbesondere damit erklären, dass mit Erreichen dieses maximalen Verdichtungszustands bei weiterem Rütteln keine weitere Verdichtung mehr erfolgt, sondern lediglich eine Korn-auf-Korn-Verspannung und/oder Kornbruch.

Der plötzliche Anstieg der Bodensteifigkeit beziehungsweise der Knick im grafischen Verlauf kann auf jede geeignete Weise ermittelt werden. Beispielsweise kann ein erster Bodensteifigkeitswert zu einem ersten Zeitpunkt und ein zweiter Bodensteifigkeitswert zu einem späteren zweiten Zeitpunkt so ermittelt werden, dass die zum zweiten Bodensteifigkeitswert zugehörige Steigerungsrate deutlich größer ist, als die erste Bodensteifigkeitssteigerungsrate. Ein zeitlicher Abstand zwischen dem ersten Zeitpunkt und dem zweiten Zeitpunkt kann in geeigneter Form gewählt werden und beispielsweise weniger als 10 % und/oder mehr als 1 % der Dauer des Verdichtungsvorgangs in einer Verdichtungstiefe sein. Aus dem ermittelten ersten und zweiten Bodensteifigkeitswert kann ein Übergangs-Bodensteifigkeitswert berechnet werden, der zwischen den genannten Werten liegt. Der Übergangswert repräsentiert den Punkt, an welchem die Bodensteifigkeit plötzlich zunimmt. Alternativ oder in Ergänzung kann der Übergangs-Bodensteifigkeitswert durch Regressionsanalyse des Bodensteifigkeitsverlaufs ermittelt werden, insbesondere durch mathematische Zerlegung in zwei Regressionsgraden. Der Schnittpunkt der Regressionsgraden kann als Übergangswert-Bodensteifigkeitswert berechnet werden. Nach einer weiteren Möglichkeit kann aus den in einer Verdichtungstiefe laufend ermittelten Bodensteifigkeiten (k) jeweils Bodensteifigkeitssteigerungsraten (k') abgeleitet werden, wobei eine erste Bodensteifigkeitssteigerungsrate (k'1) mit zumindest einer zu einem späteren Zeitpunkt ermittelten zweiten Bodensteifigkeitssteigerungsrate (k'2) verglichen und hieraus ein Übergangs-Bodensteifigkeitswerts (k12) für die jeweilige Verdichtungstiefe ermittelt werden kann, wenn die zweite Bodensteifigkeitssteigerungsrate (k'2) die erste Bodensteifigkeitssteigerungsrate (k'1) um einen definierten Faktor überschreitet.

Durch Verwendung der über die Zeit überwachten Bodensteifigkeit als Kriterium für die Beendigung des Verdichtens in einer bestimmten Tiefe, wird ein maximaler Verdichtungsgrad mit größtmöglicher Effizienz erreicht. Durch den Abbruch des Rüttelns bei Erreichen eines deutlichen Anstiegs der Bodensteifigkeit kann eine maximale Verdichtung in minimaler Rüttelzeit erreicht werden. Es wird vermieden, dass unnötige Zeit auf Rütteln ohne nennenswerten Verdichtungserfolg verwendet wird. Ferner kann der Verschleiß am Tiefenrüttler, der mit zunehmendem Verdichtungsgrades beispielsweise aufgrund von Kornverspannungen an der Rüttlerspitze zunimmt, deutlich vermindert werden.

Nach einer möglichen Konkretisierung kann das Verdichten in der jeweiligen Verdichtungstiefe insbesondere abgebrochen werden, wenn die ermittelte zweite Bodensteifigkeitssteigerungsrate größer ist als das 1,5-fache einer oder mehrerer zu einem vorherigen Zeitpunkt ermittelten Bodensteifigkeitssteigerungsraten ist, insbesondere größer als das 2,0-fache einer oder mehrerer zu einem vorherigen Zeitpunkt ermittelten Bodensteifigkeitssteigerungsraten.

Eine so ermittelte deutliche Steigerung der Bodensteifigkeit lässt zuverlässig Rückschlüsse auf die erreichte maximale oder zumindest weitestgehend erreichte Verdichtung zu.

Nach einer Verfahrensführung ist vorgesehen, dass der Tiefenrüttler zunächst bis zur gewünschten Endtiefe, gegebenenfalls unter Zuführung von Wasser, in den Boden eingerüttelt wird. Anschließend wird der Tiefenrüttler in Stufen gezogen, um den Boden von unten nach oben stufenweise zu verdichten. Dabei kann der Tiefenrüttler nach einer ersten Möglichkeit nach dem Ziehen auf die nächste Stufe in der jeweiligen Verdichtungstiefe gehalten werden. Alternativ kann der Tiefenrüttler auch unter Stopfbewegungen wieder in tiefere Bodenbereiche eingerüttelt werden, was auch als Pilgerschrittverfahren bezeichnet wird. Während des schrittweisen Verdichtens wird der die Bodensteifigkeit repräsentierende Steifigkeitskennwert k über der Tiefe der Verdichtungssäule ermittelt, so dass sich insgesamt ein Steifigkeitsprofil des Bodens über der Tiefe ergibt. Das Steifigkeitsprofil kann beispielsweise mittels eines geeigneten Datenspeichers gespeichert und für die Verdichtung weiterer Säulen eines Verdichtungsfeldes verwendet werden.

Nach einer möglichen Ausführungsform können an dem Tiefenrüttler mehrere Sensoren angebracht sein, die das Bewegungsverhalten bzw. die Beschleunigung und/oder die Lage der Unwucht sensieren. Vorzugsweise sind mehrere Beschleunigungssensoren in verschiedenen Ebenen am Tiefenrüttler angebracht. Das schrittweise Ziehen des Tiefenrüttlers kann im Verhältnis zur Lage der Ebenen der Beschleunigungssensoren insbesondere so gewählt werden, dass zumindest einer der Beschleunigungssensoren, beziehungsweise die zugehörige Ebene, einer oberen Verdichtungstiefe (T) innerhalb des beim vorherigen Verdichteten der darunterliegenden Verdichtungstiefe zwischen den Ebenen definierten Tiefenabschnitts liegt.

Aus bidirektionalen Beschleunigungsmessungen in zwei Ebenen des Rüttlers kann das horizontale Bewegungsverhalten an beliebigen Stellen des Rüttlers, beispielsweise an der Spitze oder der Lage der Unwuchtanregung ermittelt werden. Durch zweifache Integration dieser Horizontalbeschleunigungen können die zugehörigen Schwingwege ermittelt werden, welche halbiert zur Schwingweg-Amplitude A führen. Die Rüttlerbewegung hinkt der verursachenden dynamischen Anregung durch die drehend angetriebene Unwucht hinterher. Durch die Bestimmung der Lage der Unwucht und deren Vergleich mit der Rüttlerbewegung kann der Vorlaufwinkel $\varphi$ ermittelt werden.

Die Bodensteifigkeit wird insbesondere unter Berücksichtigung des Vorlaufwinkels ($\varphi$) berechnet. Der Vorlaufwinkel ($\varphi$) bezeichnet den Phasenwinkel, um welchen die Unwuchtmasse gegenüber der Messrichtung der Sensoren bei der Rüttelbewegung versetzt ist. In Ergänzung kann das die Bodensteifigkeit des Bodens repräsentierende Bodensteifigkeitssignal (k) auch unter Berücksichtigung der Masse (M) des Tiefenrüttlers berechnet werden. Alternativ oder in Ergänzung kann das Ermitteln des die Bodensteifigkeit des Bodens repräsentierenden Bodensteifigkeitssignals (k) unter Berücksichtigung eines die am Tiefenrüttler mitschwingende Bodenmasse repräsentierenden Bodenmassenkennwerts ($\Delta M$) durchgeführt werden, insbesondere der modalen mitschwingenden Bodenmasse. Dieser die mitschwingende Bodenmasse repräsentierende Bodenmassenkennwert ($\Delta M$) kann beispielsweise auf Basis der Unwucht ($m \cdot e$), der Schwingungsamplitude (A) und der Masse (M) des Tiefenrüttlers berechnet werden, wobei die Berechnung insbesondere zumindest näherungsweise gemäß der Formel:

$$\Delta M = \frac{m \cdot e}{A} - M$$

erfolgen kann.

Nach einer bevorzugten Verfahrensführung kann das Ermitteln des die Bodensteifigkeit des Bodens repräsentierenden Bodensteifigkeitssignals (k) auch unter Berücksichtigung der gemessenen Amplitude (A) und einer Vergleichsamplitude (Aoo) erfolgen. Als Vergleichsamplitude kann insbesondere die Amplitude des Rüttlers bei einer bestimmten Erregerfrequenz ($\omega$) bei freier Schwingung verwendet werden. Um die Vergrößerungsfunktion (V) zu berechnen, kann die gemessene Schwingweg-Amplitude (A) auf die theoretische Amplitude (Aoo) bei theoretisch unendlich hoher Erregerfrequenz bezogen werden, das heißt

$$V = \frac{A}{A_\infty} = \frac{A \cdot (M + \Delta M)}{m \cdot e}$$

wobei M die modale Rüttlermasse, $\Delta M$ die modale mitschwingende Bodenmasse und $m \cdot e$ die Unwucht im Rüttler (Unwuchtmasse mal Exzentrizität) ist. Bei freier Schwingung beträgt der Vergrößerungsfaktor eins.

Nach einer möglichen Verfahrensführung kann die Ermittlung des die Bodensteifigkeit des Bodens repräsentierenden Bodensteifigkeitssignals (k) zumindest näherungsweise nach der Formel:

$$k = m \cdot e \cdot \omega^2 \cdot \left( \frac{1}{A_\infty} - \frac{1}{A} \cdot \frac{\text{sign}\,(\varphi - 90°)}{\sqrt{1 + \tan^2 \varphi}} \right)$$

wobei

- A die Schwingungsamplitude des Tiefenrüttlers während des Verdichtens,
- Aoo die Schwingungsamplitude des Tiefenrüttlers bei freier Schwingung, beziehungsweise bei gegen Unendlich laufender Erregerfrequenz,
- F die Zentrifugalkraft,
- m die Unwuchtmasse,
- e die Exzentrizität der rotierenden Unwucht m zur Drehachse,
- ω die Winkelgeschwindigkeit der rotierenden Unwucht, und
- φ der Phasenvorlauf der rotierenden Unwucht m zur Masse M des Tiefenrüttlers,

sind. Mit dieser Formel wird auch der dynamische Anteil der schwingungsrelevanten Größen berücksichtigt. Es versteht sich jedoch, dass grundsätzlich auch andere Berechnungsmethoden für die Berechnung eines die Steifigkeit des Bodens repräsentierenden Steifigkeitssignals möglich sind.

Die Aufgabe wird ferner gelöst durch ein Verfahren zur Verbesserung eines Baugrundes mittels eines Tiefenrüttlers, mit den Schritten: Erstellen eines ersten Verdichtungskörpers, wobei der Tiefenrüttler in den Baugrund bis zu einer gewünschten Endtiefe in den Boden eingebracht wird, und, nach Erreichen der Endtiefe, der Tiefenrüttler in Rüttelintervallen rüttelnd aus dem Baugrund um ein definiertes Maß gezogen wird, um den Baugrund schrittweise in Tiefenabschnitten zu verdichten, wobei während des Einrüttelns in der jeweiligen Verdichtungstiefe das Verfahren nach zumindest einer der oben genannten Ausführungsformen durchgeführt wird, wobei die in den jeweiligen Verdichtungstiefen ermittelten Bodensteifigkeitsraten, bei der das Verdichten beendet wird, erfasst und als Datenpaare aus einem Tiefenwert und einem zugehörigen Bodensteifigkeitswert gespeichert werden.

Mit dem genannten Verfahren können die bei der erstmaligen online-Verdichtungserfassung gewonnenen Daten für weitere zu erstellende Verdichtungskörper verwendet werden. Hiermit können weitere Verdichtungskörper eines zu erstellenden Verdichtungsfeldes besonders schnell hergestellt werden. Durch die Erfassung und das Speichern der Bodensteifigkeit und/oder der Bodensteifigkeitsrate über der Tiefe, die im Wege des oben genannten Verfahrens zur online-Verdichtungserfassung gewonnen werden, können diese Daten für nachfolgende Verdichtungsvorgänge herangezogen werden. Das genannte Verfahren zur online-Verdichtungserfassung braucht also allenfalls bei einer Teilzahl von mehreren zu erstellenden Verdichtungssäulen durchgeführt zu werden. Weitere Verdichtungskörper können auf Basis der vorher über der Tiefe erfassten und als Abbruchkriterium für die jeweilige Tiefe verwendeten Bodensteifigkeit und/oder der Bodensteifigkeitsrate Werte hergestellt werden.

Es ist insbesondere vorgesehen, dass zumindest ein zweiter Verdichtungskörper dadurch erstellt wird, dass die beim Erstellen des ersten Verdichtungskörpers in den jeweiligen Verdichtungstiefen ermittelten Bodensteifigkeitsraten, bei der das Verdichten jeweils beendet worden ist, zur Steuerung des Verdichtungsverfahrens zur Herstellung des zweiten Verdichtungskörpers verwendet werden. Ein Verdichtungskörper kann auch als Verdichtungssäule oder Bodensäule bezeichnet werden.

Das Erstellen einer zweiten Bodensäule erfolgt vorzugsweise, wie auch das Herstellen einer ersten Säule, durch Einrütteln des Tiefenrüttlers bis auf die gewünschte Endtiefe und anschließendes schrittweises Verdichten in Teilabschnitten bis zum Erreichen der Bodenoberfläche. Die Endtiefe ist der tiefste Verdichtungspunkt, der später das untere Ende der hergestellten Verdichtungssäule bildet. Das Maß, um das der Tiefenrüttler zum stufenweisen Verdichten in Schritten von der Endtiefe bis zur Bodenoberfläche gezogen wird, kann beispielsweise jeweils zwischen 0,25 und 1,5 m betragen.

Bevorzugte Ausführungsbeispiele werden nachstehend anhand der Zeichnungsfiguren erläutert. Hierin zeigt:

Figur 1    einen beispielhaften Tiefenrüttler, der zur Durchführung des erfindungsgemäßen Verfahrens zur Verdichtungserfassung und -steuerung sowie zur Herstellung einer Verdichtungssäule geeignet ist;

Figur 2    schematisch ein Verfahren zum Herstellen einer Verdichtungssäule mit erfindungsgemäßer online-Verdichtungserfassung und -steuerung;

Figur 3    eine Anordnung mit Tiefenrüttler beim Verdichten von Boden eines zu verdichtenden Feldes mit schematisch eingezeichnetem Modell zur Beschreibung der dynamischen Verhältnisse beim Rütteln; und

Figur 4      eine grafische Darstellung von verschiedenen Kennwerten über der Zeit bei der Durchführung des erfindungsgemäßen Verfahrens in einer Tiefenstufe.

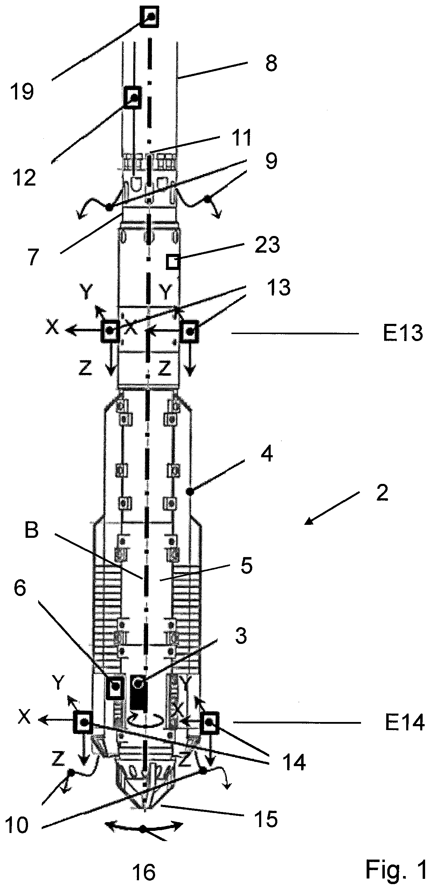

Die Figuren 1 bis 4 werden nachstehend gemeinsam beschrieben. Ein Tiefenrüttler 2 dient zum Verdichten von Boden mittels einer Unwucht. Als Unwucht wird ein rotierender Körper 3 verstanden, dessen Masse nicht rotationssymmetrisch verteilt ist. Die Massenträgheitsachse des Massekörpers 3 ist gegenüber der Rotationsachse B versetzt, so dass die Unwucht beim Rotieren Schwingungen erzeugt, mit denen das Erdreich und mögliches Zugabematerial verdichtet wird. Das Verfahren der Rütteldruckverdichtung beruht auf dem Effekt, dass durch die Vibration des Tiefenrüttlers 2 die Reibung zwischen den Bodenkörnern kurzzeitig aufgehoben wird und vorhandene Porenräume infolge der Schwerkraft nahezu bis zur dichtesten Lagerung zufallen. Je nach Bodenbeschaffenheit und Verdichtungsaufwand tritt dabei eine Volumenverminderung ein.

Ein für die Durchführung des erfindungsgemäßen Verfahrens zur Verdichtungserfassung und Verdichtungssteuerung geeigneter Tiefenrüttler 2 umfasst entsprechend als wesentliche Bestandteile den rotierend antreibbaren Massekörper 3, der in einem Rüttlergehäuse 4 um die Drehachse B drehend antreibbar ist. Der Massekörper 3 kann von einem Drehantrieb 5, beispielsweise einem Elektromotor, über eine Antriebswelle (nicht dargestellt) angetrieben werden. Ein die Lage der Unwucht 3 repräsentierendes Lagesignal kann mittels eines entsprechenden Sensors 6 erfasst werden.

Der Tiefenrüttler 2 kann über eine elastische Kupplung 7 an einem Gestänge 8 aufgehängt werden. Das Versenken und/oder das Verdichten kann optional durch eine oder mehrere Wasserspülungen 9, 10 über in dem Gestänge 8 integrierte Leitungen 11 erleichtert werden. Der Wasserdurchfluss und/oder der Wasserdruck können gegebenenfalls mittels entsprechender Sensoren 12 gemessen und danach gesteuert werden.

Es sind erste Beschleunigungsaufnehmer 13 in einer ersten Ebene E13 des Tiefenrüttlers 2 vorgesehen, insbesondere oberhalb der Unwucht 3, und zweite Beschleunigungsaufnehmer 14 in einer zweiten Ebene E14, insbesondere unterhalb der Unwucht 3. Die Beschleunigungsaufnehmer 13, 14 dienen zur Messung der Beschleunigung des Tiefenrüttlers 2 während des Rüttelvorgangs. Aus bidirektionalen Beschleunigungsmessungen in zwei Ebenen E13, E14 des Rüttlers 2 kann das horizontale Bewegungsverhalten an beliebigen Stellen des Rüttlers, beispielsweise an der Spitze 15 oder in der Lage der Anregung durch die Unwucht 3 ermittelt werden. Dabei gilt insbesondere, dass der Schwingweg 16 an der Rüttlerspitze 15 der doppelten Schwingweg-Amplitude entspricht.

Es können ferner Kraftsensoren 19 zum Erfassen der Aufhängekraft des Rüttlers 2 beziehungsweise zur Bestimmung des Spitzendruckes des Rüttlers vorgesehen sein. Außerdem kann zumindest ein Sensor 23 zur Messung der Eindringtiefe T des Tiefenrüttlers 2 vorgesehen sein.

Das erfindungsgemäße Verfahren zur Verdichtungserfassung wird während des Tiefenrüttelverfahrens durchgeführt, weswegen es auch als online-Verdichtungserfassung bezeichnet werden kann. Beim Tiefenrüttelverfahren wird der Tiefenrüttler 2 zunächst bis zur vorgesehenen Verbesserungstiefe Tm, gegebenenfalls unter Zuführung von Wasser, in den Baugrund 17 versenkt. Anschließend wird der Tiefenrüttler 2 stufenweise in niedrigere Tiefen (Tm-1, Tm-2 ... T2, T1) gezogen, um den jeweils nächsten, darüber liegenden Bodenabschnitt zu verdichten. Dabei kann der Tiefenrüttler 2 unter Stopfbewegungen wieder in tiefere Bodenbereiche eingerüttelt werden. Insgesamt wird somit ein Verdichtungskörper 18 schrittweise hergestellt. Es ist für eine möglichst genaue Verdichtungserfassung insbesondere vorgesehen, dass das Hebemaß ($\Delta$T), um das der Tiefenrüttler 2 jeweils stufenweise gezogen wird, um übereinanderliegende Verdichtungsabschnitte zu erzeugen, kleiner oder gleich dem zwischen den zwei Ebenen E13, E14 der Beschleunigungssensoren 13, 14 gebildeten Abstandsmaß. Während des schrittweisen Verdichtens wird je Verdichtungsschritt ein die Bodensteifigkeit repräsentierender Steifigkeitskennwert k über der Zeit t ermittelt. Aus einer Vielzahl einzelner über der Tiefe T des Verdichtungskörpers 18 ermittelten Steifigkeitskennwerte k, insbesondere aus über der Tiefe berechneten charakteristischen Steifigkeitskennwerten, kann insgesamt ein Steifigkeitsprofil k(T) des Bodens 17 über der Tiefe T abgeleitet werden. Das Steifigkeitsprofil k(T) kann beispielsweise mittels eines geeigneten Datenspeichers gespeichert und für die Verdichtung weiterer Säulen eines Verdichtungsfeldes verwendet werden.

Eine Besonderheit des vorliegenden Verfahrens zur Verdichtungserfassung beziehungsweise Verdichtungssteuerung ist, dass die über der Zeit laufend ermittelten Bodensteifigkeitssignale k mit zumindest einem zu einem vorherigen Zeitpunkt ermittelten Bodensteifigkeitssignals k verglichen und hieraus jeweils eine Bodensteifigkeitssteigerungsrate k' abgeleitet werden kann. Diese Steigerungsrate k' gibt an, um wieviel die Steifigkeit des Bodens 17 verglichen mit einem vorher ermittelten Wert gegebenenfalls zugenommen hat. Durch Vergleichen der aktuell ermittelten Bodensteifigkeitssteigerungsrate k' mit einer oder mehreren vorher ermittelten Steigerungsraten k' können Rückschlüsse über den Verdichtungsgrad des zu verdichtenden Bodenabschnitts gezogen werden. Insbesondere lässt sich erkennen, wenn der Anstieg der Steifigkeit des Bodens 17 sprunghaft zunimmt. Dabei ist erfindungsgemäß vorgesehen, dass das Rüttelverfahren in einer jeweiligen Verdichtungstiefe (Tm, Tm-1, ..., T1) beendet wird, wenn die aktuell ermittelte Bodensteifigkeitssteigerungsrate k' zumindest eine zu einem vorherigen Zeitpunkt ermittelte Bodensteifigkeitssteigerungsrate k' um einen vorher festgelegten Faktor überschreitet.

Die Wahl des Faktors kann unter Berücksichtigung der spezifischen technischen Anforderungen an die herzustellende Verdichtungssäule und/oder der Bodenbeschaffenheit nach Bedarf festgelegt werden. Beispielsweise kann vorgesehen sein, dass das Verdichten in der jeweiligen Verdichtungstiefe (Tm, Tm-1, ..., T1) abgebrochen wird, wenn die ermittelte Bodensteifigkeitssteigerungsrate k' größer ist als das 1,5-fache, insbesondere größer als das 2,0-fache, gegebenenfalls größer als das 5,0-fache einer oder mehrere zu einem vorherigen Zeitpunkt ermittelten Bodensteifigkeitssteigerungsraten ist. Eine derart deutliche Steigerung der Bodensteifigkeit um mindestens das 1,5-fache lässt darauf schließen, dass eine größtmögliche Verdichtung erreicht ist.

Die Berechnung des Bodensteifigkeitskennwerts k erfolgt insbesondere auf Basis von zumindest dem Vorlaufwinkel φ, um den die Unwuchtmasse 3 gegenüber der Bewegungsrichtung des Rüttlergehäuses 4 bei der Rüttelbewegung vorläuft, der modalen Masse M des Tiefenrüttlers 2, eines die am Tiefenrüttler 2 mitschwingende Bodenmasse repräsentierenden Bodenmassenkennwerts ΔM und der Schwingungsamplitude A des Tiefenrüttlers 2. Die Rüttlermasse M kann durch Amplitudenmessung am Tiefenrüttler 2 vor dem Eindringen in den Boden 17 bestimmt werden. Die Bestimmung der modalen Gesamtmasse aus Rüttlermasse M und modaler mitschwingender Bodenmasse ΔM kann im eingerüttelten Zustand während des Betriebs, insbesondere in Betriebsphasen mit einem Vorlaufaufwinkel φ von annähernd 180°, womit ein Bereich von 180° ± 10° umfasst sein soll, bestimmt werden.

Vorzugsweise wird das die Bodensteifigkeit des Bodens 17 repräsentierende Bodensteifigkeitssignals k unter Berücksichtigung eines Vergrößerungsfaktors V beziehungsweise einer Vergleichsamplitude ermittelt. Als Vergleichsamplitude kann die Amplitude des Rüttlers 2 bei einer bestimmten Erregerfrequenz ω bei freier Schwingung verwendet werden. Insbesondere wird die gemessene Schwingweg-Amplitude A auf die Amplitude bei theoretisch unendlich hoher Erregerfrequenz Aoo bezogen, um den Vergrößerungsfaktor V zu berechnen:

$$V = \frac{A}{A_\infty} = \frac{A \cdot (M + \Delta M)}{m \cdot e}$$

Die dynamische Bodensteifigkeit k kann dann aus den gemessenen Größen und der Erregerkreisfrequenz ω insbesondere mit Hilfe der folgenden Formel ermittelt werden:

$$k = m \cdot e \cdot \omega^2 \cdot \left( \frac{1}{A_\infty} - \frac{1}{A} \cdot \frac{\text{sign}\,(\varphi - 90°)}{\sqrt{1 + \tan^2 \varphi}} \right)$$

Mit dem Frequenzverhältnis β

$$\beta = \left( 1 - \text{sign}(\varphi - 90°) \cdot \left( \frac{A_\infty}{A} \right) \cdot (1 + \tan^2 \varphi)^{-\frac{1}{2}} \right)^{-\frac{1}{2}}$$

kann auch das zugehörige Lehrsche Dämpfungsmaß D ermittelt werden:

$$D = tan \left( \varphi - \frac{\text{sign}(\varphi - 90°) + 1}{2} \cdot 180° \right) \cdot \frac{1 - \beta^2}{2 \cdot \beta}$$

Auf diese Weise ist es möglich, laufend die zustandsabhängige Bodensteifigkeit und die dämpfende Wirkung des Bodens 17 während des Eindringens des Tiefenrüttlers 2 in den Boden beziehungsweise während des Verdichtungsprozesses zu ermitteln.

Es versteht sich, dass die Berechnung eines die Bodensteifigkeit repräsentierenden Kennwertes nicht auf die beschriebene Möglichkeit beschränkt ist, sondern, dass prinzipiell auch andere Berechnungsmethoden zur Bestimmung des Bodensteifigkeitskennwertes zum Einsatz kommen können.

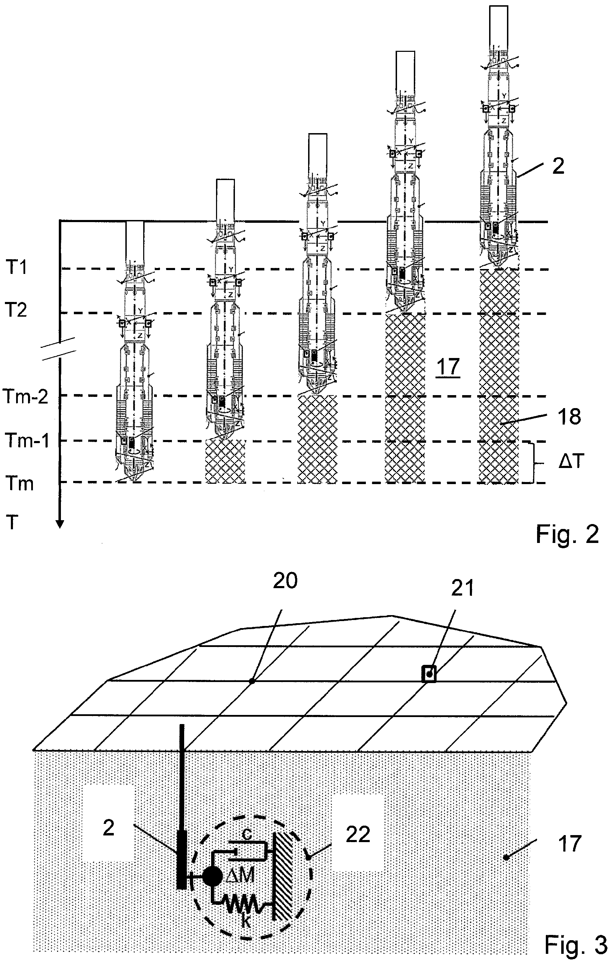

Als Grundlage zur Ermittlung der Bodensteifigkeit kann für das Rüttelverfahren mittels eines Tiefenrüttlers 2 das mechanische Modell eines harmonisch angeregten Feder-Dämpfer-Systems 22 mit herangezogen werden, wie es in Figur 3 gezeigt ist. Dabei ist ΔM die mitschwingende modale Bodenmasse, k die Federsteifigkeit beziehungsweise Bodensteifigkeit und c ein Dämpfungskoeffizient. Es sind ferner ein Verdichtungsraster 20 auf der Bodenoberfläche erkennbar sowie ein Sensor 21 zur Messung von Oberflächenwellen. Messungen von Oberflächenwellen am Boden um einen Verdichtungspunkt können Aufschlüsse über das dynamische Rüttler-Boden-Interaktionssystem 22 geben.

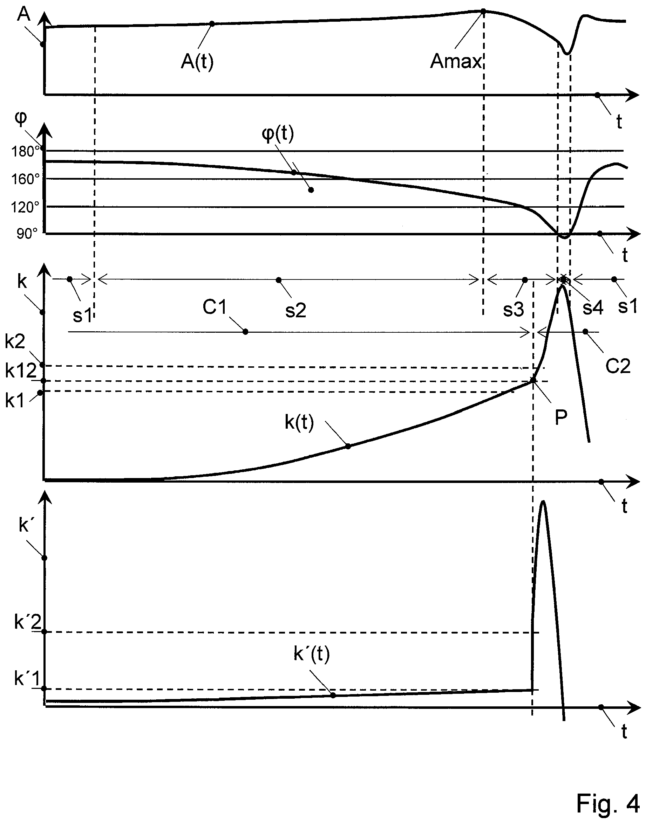

Figur 4 zeigt verschiedene Kennwerte beim Verdichten mittels eines Tiefenrüttlers 2 über der Zeit für einen beispielhaften Verdichtungsvorgang in einem Verdichtungsabschnitt. Der obere Graph zeigt die Schwingungsamplitude A, der zweite Graph den Vorlaufwinkel φ, der dritte Graph die Bodensteifigkeit k und der vierte Graph die Ableitung k' der Bodensteifigkeit, jeweils über der Zeit t beziehungsweise in verschiedenen Betriebsphasen s1, s2, s3. Dabei bezeichnet s1 die Betriebsphase des Hebens (Ziehens) des Tiefenrüttlers 2 von einer tieferliegenden Tiefe ™ auf die aktuelle Tiefe (Tm-1). Im nachfolgenden Schritt, welcher mit s2 bezeichnet ist, findet das Einrütteln in den zu verdichtenden Bodenabschnitt statt. Dabei erfolgt eine Verdichtung des Bodens 17 durch Kornrumlagerung, wobei eine bleibende Steifigkeitserhöhung durch eine Verringerung des Porenvolumens im Boden 17 erreicht wird. Es ist erkennbar, dass der Bodensteifigkeitskennwert k über der Zeit t während des Einrüttelns und Verdichtens in der Betriebsphase s2 im Wesentlichen stetig ansteigt. Das heißt, die Steigung des Bodensteifigkeitskennwerts k über der Zeit ist nahezu konstant beziehungsweise nimmt leicht zu.

Mit zunehmender Verdichtung steigt die Schwingungsamplitude A des Tiefenrüttlers 2 leicht an, bis ein Amplitudenmaximum Amax bei Resonanz erreicht ist. Ab diesem Punkt liegt die Betriebsphase s3 Postresonanz vor. Es erfolgt eine weitere Erhöhung der Bodensteifigkeit k mit im Wesentlichen konstanter Steigung, solange der Boden 17 durch Kornumlagerung weiter verdichtet wird. Ist eine maximale Verdichtung erreicht, steigt die Bodensteifigkeit k(t) in einem Knick an, der hier als Punkt P eingezeichnet ist. Der zugehörige Bodensteifigkeits-Übergangswert k12, liegt zwischen einem ersten Bodensteifigkeitswert k1 mit kleinerer Steigung und einem größeren Bodensteifigkeitswert k2 mit größerer Steigung. Entsprechend dem Knick im Punkt P der Kurve k(t) steigt die Ableitung beziehungsweise Steigungsrate k' der Bodensteifigkeit hier sprunghaft an. Dieser Knickpunkt P markiert den Übergang vom Verdichten C1 durch Kornumlagerung hin zur Kornverspannung C2, die eine temporäre Steifigkeitserhöhung durch die momentan erhöhte Vertikalbelastung des Bodens 17 durch den Rüttler 2 verursacht. Diese temporäre Steifigkeitserhöhung des Bodens 17 führt zu einer deutlich größeren Steigung des berechneten Bodensteifigkeitskennwerts k ab diesem Übergangspunkt P. Nachfolgend steigt der Bodensteifigkeitskennwert k mit nahezu konstanter Steigung weiter an, bis ein "Überdrücken" in der Betriebsphase s4 erreicht wird. Im daran anschließenden Schritt s1 wird der Rüttler 2 wieder gehoben, so dass der Steifigkeitskennwert k wieder abfällt.

Es ist weiter erkennbar, dass der Vorlaufwinkel φ während des Verdichtens C langsam abfällt. Dabei liegt der Vorlaufwinkel φ bei beginnender Verdichtung knapp unter 180° und sinkt bis zum Erreichen des Übergangspunkts P auf etwa 120°. Ab dem Erreichen des Übergangs-Bodensteifigkeitswerts k12 fällt der Vorlaufwinkel φ stärker ab, bis auf etwa 90°, die im überdrückten Zustand anliegen. Beim erneuten Heben s1 steigt der Phasenwinkel wieder auf den Ausgangswert an.

Gemäß dem vorliegenden Verfahren ist vorgesehen, dass das Erreichen des Übergangs-Bodensteifigkeitswerts k12 beziehungsweise der sprunghafte Anstieg der Steigung des Bodensteifigkeitskennwerts k als Abbruchkriterium für das Beenden des Rüttelvorgangs in der jeweiligen Verdichtungstiefe verwendet wird. Hiermit wird ein besonders effizientes Verdichtungsverfahren erreicht, da einerseits eine zu geringe Verdichtung und andererseits ein zu langes Verweilen in einer Verdichtungsstufe ohne zusätzlichen Verdichtungserfolg vermieden werden.

Bezugszeichenliste

| | |
|---|---|
| 2 | Tiefenrüttler |
| 3 | Massekörper |
| 4 | Rüttlergehäuse |
| 5 | Drehantrieb |
| 6 | Sensor / Winkelgeber |
| 7 | Kupplung |
| 8 | Gestänge |
| 9 | Wasserspülung |
| 10 | Wasserspülung |
| 11 | Leitung |
| 12 | Sensor / Wasserdruckgeber, Wasserdurchflussmengenzähler |
| 13 | Sensor / Beschleunigungsaufnehmer |
| 14 | Sensor / Beschleunigungsaufnehmer |
| 15 | Spitze |
| 16 | Schwingweg |
| 17 | Boden |
| 18 | Verdichtungskörper |
| 19 | Kraftsensor |
| 20 | Verdichtungsraster |

| 21 | Sensor / Beschleunigungsaufnehmer |
| 22 | Feder-Dämpfer-System mit modal mitschwingender Bodenmasse |
| 23 | Sensor / Wegaufnehmer |

| A | Amplitude |
| Aoo | Amplitude bei unendlich hoher Erregerfrequenz |
| B | Drehachse |
| C1 | Verdichtung, Kornumlagerung |
| C2 | Kornverspannung |
| c | Dämpfungskoeffizient |
| e | Exzenter der Unwucht |
| E | Ebene |
| k | Bodensteifigkeitskennwert |
| k' | Bodensteifigkeitssteigerungsrate |
| s1, s2, s3 | Betriebsphasen |
| t | Zeit |
| T | Tiefe |
| m | Masse der Unwucht |
| M | modale Rüttlermasse |
| ΔM | modale mitschwingende Bodenmasse |
| P | Übergangspunkt |
| V | Vergrößerungsfaktor |
| β | Frequenzverhältnis |
| φ | Vorlaufwinkel |
| ω | Erregerfrequenz |

**Patentansprüche**

**1.** Verfahren zur Verdichtungserfassung und -steuerung beim Verdichten eines Bodens mittels eines Tiefenrüttlers, wobei der Tiefenrüttler (2) eine in einem Rüttlergehäuse (4) drehend antreibbare Unwucht (3) sowie zumindest einen Sensor (6, 12, 13, 14, 19) aufweist, mit den Schritten:

Einbringen des Tiefenrüttlers (2) in den Boden (17) bis zu einer gewünschten Endtiefe (Tm);
Verdichten des Bodens (17) mittels des Tiefenrüttlers (2) in Verdichtungsschritten, wobei während des Verdichtens zu einer jeweils gemessenen Tiefe (T) der Vorlaufwinkel (φ) der Unwucht (3) sowie die Schwingungsamplitude (A) des Tiefenrüttlers (2) ermittelt werden;
während eines Verdichtungsschritts, Erfassen eines Bodensteifigkeitsverlaufs aus über der Zeit (t) auf Basis zumindest des Vorlaufwinkels (φ) und der Schwingungsamplitude (A) ermittelten Bodensteifigkeitswerten (k);
Ermitteln eines ersten Bodensteifigkeitswerts (k1) und eines zweiten Bodensteifigkeitswerts (k2) aus dem Bodensteifigkeitsverlauf, für die gilt, dass eine Steigerungsrate (k'2) des zweiten Bodensteifigkeitswerts (k2) eine Steigerungsrate (k'1) des ersten Bodensteifigkeitswerts (k1) um einen definierten Faktor überschreitet;
Berechnen eines Übergangs-Bodensteifigkeitswerts (k12), der zwischen dem ersten Bodensteifigkeitswert (k1) und dem zweiten Bodensteifigkeitswert (k2) liegt;
Speichern des in dem jeweiligen Verdichtungsschritt erfassten Übergangs-Bodensteifigkeitswerts (k12) zur zugehörigen Tiefe (T).

**2.** Verfahren nach Anspruch 1,
**dadurch gekennzeichnet,**
**dass** das Verdichten in einem Verdichtungsschritt beendet wird, wenn die Steigerungsrate (k'2) des zweiten Bodensteifigkeitswerts (k2) größer ist als das 1,5-fache der Steigerungsrate (k'1) des ersten Bodensteifigkeitswerts (k1), insbesondere größer als das 2-fache der Steigerungsrate (k'1) des ersten Bodensteifigkeitswerts (k1) ist.

**3.** Verfahren nach Anspruch 1 oder 2,
**dadurch gekennzeichnet,**
**dass** der Tiefenrüttler (2) nach Beendigung des Verdichtens in einer Verdichtungstiefe (T) zur nächsten zu verdichtenden Tiefe gezogen wird, um den Boden in dieser nächsten Tiefe zu verdichten.

**4.** Verfahren nach einem der Anspruche 1 bis 3,
**dadurch gekennzeichnet,**
**dass** mindestens ein Beschleunigungssensor (13, 14) am Tiefenrüttler (2) angebracht ist, der Beschleunigungen des Tiefenrüttlers (2) während des Verdichtens misst, und
ein Positionssensor (6), der ein die Position der Unwucht (3) repräsentierendes Signal erfasst.

**5.** Verfahren nach einem der Anspruche 1 bis 4,
**dadurch gekennzeichnet,**
**dass** mehrere Beschleunigungssensoren (13, 14) in verschiedenen Ebenen (E13, E14) am Tiefenrüttler (2) angebracht sind.

**6.** Verfahren nach einem der Ansprüche 1 bis 5,
**dadurch gekennzeichnet,**
**dass** das Ermitteln des die Bodensteifigkeit des Bodens (17) repräsentierenden Bodensteifigkeitswerts (k) unter Berücksichtigung einer modalen mitschwingenden Bodenmasse (ΔM) erfolgt.

**7.** Verfahren nach Anspruch 6,
**dadurch gekennzeichnet,**
**dass** die modale mitschwingende Bodenmasse (ΔM) auf Basis der Unwucht (m · e), der Schwingungsamplitude (A) und der Masse (M) des Tiefenrüttlers erfolgt, insbesondere zumindest näherungsweise gemäß der Formel:

$$\Delta M = \frac{m \cdot e}{A} - M$$

**8.** Verfahren nach einem der Ansprüche 1 bis 7,
**dadurch gekennzeichnet,**
**dass** das Ermitteln des die Bodensteifigkeit des Bodens (17) repräsentierenden Bodensteifigkeitswerts (k) auf Basis der gemessenen Amplitude (A) und einer Vergleichsamplitude (Aoo) erfolgt, wobei als Vergleichsamplitude insbesondere die Amplitude des Rüttlers bei einer beliebigen Erregerfrequenz (ω) bei freier Schwingung verwendet wird.

**9.** Verfahren nach einem der Ansprüche 1 bis 8,
**dadurch gekennzeichnet,**
**dass** die Berechnung des die Bodensteifigkeit des Bodens (17) repräsentierenden Bodensteifigkeitswerts (k) unter Berücksichtigung des momentanen Vergrößerungsfaktors (V) aus der gemessenen Amplitude (A) zur Vergleichsamplitude (Aoo) erfolgt, insbesondere nach der Formel:

$$V = \frac{A}{A_\infty} = \frac{A \cdot (M + \Delta M)}{m \cdot e}$$

wobei

- V der Vergrößerungsfaktor,
- A die Schwingungsamplitude des Tiefenrüttlers während des Verdichtens,
- Aoo die Schwingungsamplitude des Tiefenrüttlers bei freier Schwingung, beziehungsweise bei gegen Unendlich laufender Erregerfrequenz,
- M die Masse des Tiefenrüttlers,
- ΔM die modale mitschwingende Bodenmasse,
- m die Unwuchtmasse,
- e die Exzentrizität der rotierenden Unwucht m zur Drehachse
sind.

**10.** Verfahren nach einem der Ansprüche 1 bis 9,
**dadurch gekennzeichnet**,
das der Vorlaufwinkel (φ) der Unwucht (3) gegenüber der Rüttlerbewegung auf einen Wert von größer 90° und kleiner als 180° eingestellt wird, insbesondere größer 100° und kleiner 170°.

**11.** Verfahren nach einem der Ansprüche 1 bis 10,
**dadurch gekennzeichnet,**
**dass** die Ermittlung des die Bodensteifigkeit des Bodens (17) repräsentierenden Bodensteifigkeitswerts (k) zumindest näherungsweise nach der Formel:

$$k = m \cdot e \cdot \omega^2 \cdot \left( \frac{1}{A_\infty} - \frac{1}{A} \cdot \frac{\text{sign}\,(\varphi - 90°)}{\sqrt{1 + \tan^2 \varphi}} \right)$$

erfolgt, wobei

- A die Schwingungsamplitude des Tiefenrüttlers während des Verdichtens,
- Aoo die Schwingungsamplitude des Tiefenrüttlers bei freier Schwingung, beziehungsweise bei gegen Unendlich laufender Erregerfrequenz,
- m die Unwuchtmasse,
- e die Exzentrizität der rotierenden Unwucht m zur Drehachse,
- ω die Kreisfrequenz der rotierenden Unwucht, und
- φ der Phasenvorlauf der rotierenden Unwucht m zur Bewegung der Masse M des Tiefenrüttlers,

sind.

**12.** Verfahren zur Verbesserung eines Baugrundes mittels eines Tiefenrüttlers, mit den Schritten:

Erstellen eines ersten Verdichtungskörpers (18), wobei der Tiefenrüttler (2) in den Baugrund (17) bis zu einer gewünschten Endtiefe (Tm) in den Boden eingebracht wird, und
nach Erreichen der Endtiefe (Tm), der Tiefenrüttler (2) in Rüttelintervallen rüttelnd aus dem Baugrund (17) jeweils um ein definiertes Maß (ΔT) gezogen wird, um den Baugrund (17) schrittweise in Tiefenabschnitten zu verdichten,
**dadurch gekennzeichnet, dass** während des Einrüttelns in der jeweiligen Verdichtungstiefe das Verfahren nach einem der Ansprüche 1 bis 11 durchgeführt wird.

**13.** Verfahren nach Anspruch 12,
**dadurch gekennzeichnet,**
**dass** das aus den über der Tiefe (T) erfassten Übergangs-Bodensteifigkeitswerte (k12) abgeleitete Bodensteifigkeitsprofil zur Steuerung bei der Herstellung zumindest eines weiteren Verdichtungskörpers verwendet wird.

**14.** Verfahren nach Anspruch 13,
**dadurch gekennzeichnet,**
**dass** die Steuerung des Rüttelverfahrens unter Berücksichtigung der Übergangs-Bodensteifigkeitswerte (k12) derart erfolgt, dass das Verdichten in einer Verdichtungstiefe (Tm, Tm-1, ... T2, T1) beendet wird, wenn der zur jeweiligen Verdichtungstiefe zugehörige Übergangs-Bodensteifigkeitswert (k12) zumindest erreicht ist.

**15.** Verfahren nach einem der Ansprüche 12 bis 14,
**dadurch gekennzeichnet,**
**dass** das ermittelte Bodensteifigkeitsprofil zur Rasteroptimierung eines Rasters einer Mehrzahl von zu erstellenden Verdichtungskörpern (18) verwendet wird.

## Claims

**1.** Method for detecting and controlling compaction when compacting a soil by means of a depth vibrator, wherein the depth vibrator (2) has an unbalance (3) rotationally driveable in a vibrator housing (4) and at least one sensor (6, 12, 13, 14, 19), comprising the steps:

penetrating the depth vibrator (2) into the soil (17) to a desired final depth (Tm);
compacting the soil (17) by the depth vibrator (2) in compaction steps wherein, during compacting, the advance angle (φ) of the unbalance (3) and the vibration amplitude (A) of the depth vibrator (2) are determined for a respectively measured depth (T);

during a compaction step, detecting a soil stiffness profile from soil stiffness values (k) determined over time (t) on the basis of at least the advance angle (φ) and the vibration amplitude (A);

determining a first soil stiffness value (k1) and a second soil stiffness value (k2) from the soil stiffness profile, for which it applies that a rate of increase (k'2) of the second soil stiffness value (k2) exceeds a rate of increase (k'1) of the first soil stiffness value (k1) by a defined factor;

calculating a transition soil stiffness value (k12) which is between said first soil stiffness value (k1) and said second soil stiffness value (k2);

saving the transition soil stiffness value (k12) determined in the respective compaction step to the corresponding depth (T).

2. Method according to claim 1,
**characterized in**
**that** compaction is terminated in a compaction step if the rate of increase (k'2) of the second soil stiffness value (k2) is greater than 1.5 times the rate of increase (k'1) of the first soil stiffness value (k1), in particular if it is greater than twice the rate of increase (k'1) of the first soil stiffness value (k1).

3. Method according to claim 1 or 2,
**characterized in**
**that** after completion of compaction in a compaction depth (T) the depth vibrator (2) is pulled to the next depth to be compacted in order to compact the soil in this next depth.

4. Method according to any of claims 1 to 3,
**characterized in**
**that** at least one acceleration sensor (13, 14) which measures accelerations of the depth vibrator (2) during compaction, and
a position sensor (6) which detects a signal representing the position of the unbalance (3) are provided at the depth vibrator (2).

5. Method according to any of claims 1 to 4,
**characterized in**
**that** a plurality of acceleration sensors (13, 14) are attached to the depth vibrator (2) in different planes (E13, E14).

6. Method according to any of claims 1 to 5,
**characterized in**
**that** the soil stiffness value (k) representing the soil stiffness of the soil (17) is determined taking into account a modal resonating soil mass (ΔM).

7. Method according to claim 6,
**characterized in**
**that** the modal resonating soil mass (ΔM) is determined based on the unbalance (m · e), the vibration amplitude (A) and the mass (M) of the depth vibrator, in particular at least approximately according the formula:

$$\Delta M = \frac{m \cdot e}{A} - M$$

8. Method according to any of claims 1 to 7,
**characterized in**
**that** the soil stiffness value (k) representing the soil stiffness of the soil (17) is determined on the basis of the measured amplitude (A) and a reference amplitude (Aoo), wherein in particular the amplitude of the vibrator at any excitation frequency (ω) while free oscillation is used as the reference amplitude.

9. Method according to any of claims 1 to 8,
**characterized in**
**that** the soil stiffness value (k) representing the soil stiffness of the soil (17) is calculated taking into account the instantaneous amplification factor (V) of the measured amplitude (A) to the reference amplitude (Aoo), in particular according to the formula:

$$V = \frac{A}{A_\infty} = \frac{A \cdot (M + \Delta M)}{m \cdot e}$$

wherein

- V is the amplification factor,
- A is the vibration amplitude of the depth vibrator during compaction,
- Aoo is the vibration amplitude of the depth vibrator while free oscillation and/or with an excitation frequency towards infinity,
- M is the mass of the depth vibrator,
- $\Delta M$ is the modal resonating soil mass,
- m is the unbalance mass,
- e is the eccentricity of the rotating unbalance m relative to the rotary axis.

10. Method according to any of claims 1 to 9,
**characterized in**
**that** the advance angle ($\varphi$) of the unbalance (3) relative to the vibrator motion is set to a value greater than 90° and less than 180°, in particular greater than 100° and less than 170°.

11. Method according to any of claims 1 to 10,
**characterized in**
**that** the soil stiffness value (k) representing the soil stiffness of the soil (17) is determined at least approximately according to the formula:

$$k = m \cdot e \cdot \omega^2 \cdot \left( \frac{1}{A_\infty} - \frac{1}{A} \cdot \frac{\text{sign}\,(\varphi - 90°)}{\sqrt{1 + \tan^2 \varphi}} \right)$$

wherein

- A is the vibration amplitude of the depth vibrator during compaction,
- Aoo is the vibration amplitude of the depth vibrator while free oscillation and/or with an excitation frequency towards infinity,
- m is the unbalance mass,
- e is the eccentricity of the rotating unbalance m relative to the axis of rotation,
- $\omega$ is the angular frequency of the rotating unbalance, and
- $\varphi$ is the phase advance of the rotating unbalance m relative to motion of the mass M of the depth vibrator.

12. Method for improving a ground by a depth vibrator, comprising the steps:

producing a first compaction body (18), wherein the depth vibrator (2) is inserted into the soil (17) to a desired final depth (Tm), and
after reaching the final depth (Tm) the depth vibrator (2) is vibratingly pulled out of the soil (17) in vibration intervals of a defined amount ($\Delta T$) in each case in order to compact the ground (17) step by step in depth sections, **characterized in that**, during vibrating in the respective compaction depth, the method according to any one of claims 1 to 11 is carried out.

13. Method according to claim 12,
**characterized in**
**that** the soil stiffness profile derived from the transition soil stiffness values (k12) determined over the depth (T) is used to control the production of at least one further compaction body.

14. Method according to claim 13,
**characterized in**
**that** the vibration method is controlled taking into account the transition soil stiffness values (k12) in such a way that in a respective compaction depth (Tm, Tm-1, ... T2, T1) compaction is terminated when the transition soil stiffness value (k12) associated with the respective compaction depth is at least reached.

**15.** Method according to any of claims 12 to 14,
**characterized in**
**that** the determined soil stiffness profile is used for grid optimization of a grid of a plurality of compaction bodies (18) to be constructed.

**Revendications**

**1.** Procédé pour la détection et la commande de compactage lors du compactage d'un sol à l'aide d'un vibreur en profondeur, dans lequel le vibreur en profondeur (2) présente un déséquilibre (3) apte à être entraîné de façon rotative dans un boîtier de vibreur (4) ainsi qu'au moins un capteur (6, 12, 13, 14, 19), comprenant les étapes suivantes :

introduction du vibreur en profondeur (2) dans le sol (17) jusqu'à une profondeur finale (Tm) souhaitée ;
compactage du sol (17) à l'aide du vibreur en profondeur (2) par étapes de compactage, dans lequel l'angle d'avance ($\varphi$) du déséquilibre (3) ainsi que l'amplitude d'oscillation (A) du vibreur en profondeur (2) sont déterminés pendant le compactage à une profondeur (T) respectivement mesurée ;
pendant une étape de compactage, détection d'une évolution de la rigidité du sol à partir de valeurs de rigidité du sol (k) déterminées sur le temps (t) au moins sur la base de l'angle d'avance ($\varphi$) et de l'amplitude d'oscillation (A) ;
détermination d'une première valeur de rigidité du sol (k1) et d'une deuxième valeur de rigidité du sol (k2) à partir de l'évolution de la rigidité du sol, auxquelles il s'applique qu'un taux de progression (k'2) de la deuxième valeur de rigidité du sol (k2) dépasse un taux de progression (k'1) de la première valeur de rigidité du sol (k1) selon un facteur défini ;
calcul d'une valeur de rigidité du sol de transition (k12) située entre la première valeur de rigidité du sol (k1) et la deuxième valeur de rigidité du sol (k2) ;
enregistrement de la valeur de rigidité du sol de transition (k12) détectée à l'étape de compactage respective à la profondeur (T) correspondante.

**2.** Procédé selon la revendication 1,
**caractérisé en ce que**
le compactage est terminé dans une étape de compactage lorsque le taux de progression (k'2) de la deuxième valeur de rigidité du sol (k2) est supérieur à 1,5 fois le taux de progression (k'1) de la première valeur de rigidité du sol (k1), en particulier supérieur à 2 fois le taux de progression (k'1) de la première valeur de rigidité du sol (k1).

**3.** Procédé selon la revendication 1 ou 2,
**caractérisé en ce que**
à la fin du compactage à une profondeur de compactage (T), le vibreur en profondeur (2) est tiré vers la profondeur à compacter suivante, pour compacter le sol à cette profondeur suivante.

**4.** Procédé selon l'une des revendications 1 à 3,
**caractérisé par**
au moins un capteur d'accélération (13, 14) fixé au vibreur en profondeur (2), lequel mesure des accélérations du vibreur en profondeur (2) pendant le compactage, et
un capteur de position (6) détectant un signal représentant la position du déséquilibre (3).

**5.** Procédé selon l'une des revendications 1 à 4,
**caractérisé en ce que**
plusieurs capteurs d'accélération (13, 14) sont fixés à différents niveaux (E13, E14) sur le vibreur en profondeur (2).

**6.** Procédé selon l'une des revendications 1 à 5,
**caractérisé en ce que**
la détermination de la valeur de rigidité du sol (k) représentant la rigidité du sol (17) est effectuée en tenant compte d'une masse de sol résonante modale ($\Delta M$).

**7.** Procédé selon la revendication 6,

**caractérisé en ce que**

la masse de sol résonante modale (∆M) se fonde sur le déséquilibre (m · e), l'amplitude d'oscillation (A) et la masse (M) du vibreur en profondeur, en particulier au moins approximativement selon la formule suivante :

$$\Delta M = \frac{m \cdot e}{A} - M$$

8. Procédé selon l'une des revendications 1 à 7,

    **caractérisé en ce que**

    la détermination de la valeur de rigidité du sol (k) représentant la rigidité du sol (17) est effectuée sur la base de l'amplitude mesurée (A) et d'une amplitude de comparaison (A∞), dans lequel l'amplitude du vibreur à une fréquence d'excitation (ω) quelconque en oscillation libre est utilisée comme amplitude de comparaison.

9. Procédé selon l'une des revendications 1 à 8,

    **caractérisé en ce que**

    le calcul de la valeur de rigidité du sol (k) représentant la rigidité du sol (17) est effectué en tenant compte du facteur d'accroissement momentané (V) de l'amplitude mesurée (A) à l'amplitude de comparaison (A∞), en particulier selon la formule suivante :

$$V = \frac{A}{A_\infty} = \frac{A \cdot (M + \Delta M)}{m \cdot e}$$

où

- V est le facteur d'accroissement,
- A est l'amplitude d'oscillation du vibreur en profondeur pendant le compactage,
- A∞ est l'amplitude d'oscillation du vibreur en profondeur en oscillation libre,
- M est la masse du vibreur en profondeur,
- ∆M est la masse de sol résonante modale,
- m est la masse de déséquilibre,
- e est l'excentricité du déséquilibre en rotation m par rapport à l'axe de rotation.

10. Procédé selon l'une des revendications 1 à 9,

    **caractérisé en ce que**

    l'angle d'avance (φ) du déséquilibre (3) par rapport au mouvement du vibreur est réglé sur une valeur supérieure à 90° et inférieure à 180°, en particulier supérieure à 100° et inférieure à 170°.

11. Procédé selon l'une des revendications 1 à 10,

    **caractérisé en ce que**

    la détermination de la valeur de rigidité du sol (k) représentant la rigidité du sol (17) est effectuée au moins approximativement selon la formule suivante :

$$k = m \cdot e \cdot \omega^2 \cdot \left( \frac{1}{A_\infty} - \frac{1}{A} \cdot \frac{\text{sign}(\varphi - 90°)}{\sqrt{1 + \tan^2 \varphi}} \right)$$

où

- A est l'amplitude d'oscillation du vibreur en profondeur pendant le compactage,
- A∞ est l'amplitude d'oscillation du vibreur en profondeur en oscillation libre, ou à une fréquence d'excitation tendant vers l'infini,
- m est la masse de déséquilibre,
- e est l'excentricité du déséquilibre en rotation m par rapport à l'axe de rotation,
- ω est la fréquence circulaire du déséquilibre en rotation, et

- φ est l'avance de phase du déséquilibre en rotation m par rapport au mouvement de la masse M du vibreur en profondeur.

12. Procédé pour l'amélioration d'un terrain à l'aide d'un vibreur en profondeur, comprenant les étapes suivantes :

production d'un premier corps de compactage (18), le vibreur en profondeur (2) étant introduit dans le terrain (17) jusqu'à une profondeur finale (Tm) souhaitée, et
après atteinte de la profondeur finale (Tm), le vibreur en profondeur (2) est retiré sous vibration respectivement selon une mesure définie (ΔT) hors du terrain (17) par intervalles de vibration, pour compacter le terrain (17) par étapes dans des sections de profondeur,
**caractérisé par** la mise en œuvre du procédé selon l'une des revendications 1 à 11 pendant l'introduction à la profondeur de compactage respective.

13. Procédé selon la revendication 12,
**caractérisé en ce que**
le profil de rigidité du sol dérivé à partir des valeurs de rigidité du sol de transition (k12) détectées sur la profondeur (T) est utilisé pour la commande lors de la fabrication d'au moins un corps de compactage supplémentaire.

14. Procédé selon la revendication 13,
**caractérisé en ce que**
la commande du procédé par vibration est effectuée en tenant compte des valeurs de rigidité du sol de transition (k12), de telle façon que le compactage est terminé à une profondeur de compactage (Tm, Tm-1, ... T2, T1), lorsque la valeur de rigidité du sol de transition (k12) correspondant à la profondeur respective est au moins atteinte.

15. Procédé selon l'une des revendications 12 à 14,
**caractérisé en ce que**
le profil de rigidité du sol déterminé est utilisé pour l'optimisation d'un réseau constitué d'une pluralité de corps de compactage (18) à mettre en place.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012171527 A1
- DE 19928692 C1
- DE 10146342 A1
- EP 1516961 B1
- DE 102014019139 A1